# EUROPEAN PATENT APPLICATION

(11) **EP 1 029 510 A2**
(43) Date of publication of application: **23.08.2000**
(21) Application number: 00500022.9
(22) Date of filing: 11.02.2000
(51) Int. Cl.: A61B 17/70, A61F 2/44

(54) **Box-plate for stabilizing the cervical column**

(30) Priority: 11.02.1999 ES 9900286
(71) Applicant: Darder, Jose Manuel Darder, Castellon (ES)
(72) Inventor: Darder, Jose Manuel Darder, Castellon (ES)
(74) Representative: Lanza Murciano, Marino Manuel

(57) **Abstract**

Box-plate for stabilizing the cervical column, consisting of a titanium alloy part(1) presenting a rectangular base (2), provided with two perforations (4) and (4') for receiving screws (6) and (6') to be coupled to vertebral bodies (10) and (10'), the plate (2) presenting at one of its faces a projection (4) having a hollow zone (5), into which a bony graft or other osteoinhabitable material is incorporated, having, at the opposite face, a groove (7) for incorporating a fastening element or pinces for handling the plate and the support or box (1).

## Description

### BACKGROUND OF THE INVENTION

The present specification refers to an application for a Patent of Invention in relation to a box-plate for stabilizing the cervical column, the purpose of which is to be configured like an element particularly designed to prevent and surgically treat the instability of the cervical column developed after a discectomy or corporectomy performed frontally or frontal-laterally.

### FIELD OF THE INVENTION

The present invention will find application in the industry dedicated to the manufacture of apparatus, devices and auxiliary elements for surgery and surgical treatments.

### RELATED ART

Up till now, once a cervical vertebral disk or a cervical vertebral body has been removed, the best way of attaining an immediate stability of the segment is to use two - independent elements specifically configured as a simple metal plate screwed on to the vertebral bodies, and a bony or synthetic graft incorporated into the emptied disk space.

This arrangement lacks an initial stability in relation to the compression, thereby the loads are transferred thru the screws and plate, giving rise to a probable existence of a mechanical failure of these elements when the inserted bony graft collapses.

Said collapse is at least 20% the height at best, and determines the mechanical overload of the screws at the fixation point to the plate, breaking them, or, at least, putting them out of order.

If, at this time, the bony fusion has not been performed, the fitting-up fails, and, consequently, an inestability is produced.

An evident solution to the present problems in this matter would be to rely on an element, which configured like a plate or support or box would allow it to be stabilized after performing a surgical operation, discectomy or corporeotomy of the cervical column.

Nevertheless, the applicant is not aware of the existence at present of an invention fitted with the characteristics above pointed out as suitable.

### SUMMARY OF THE INVENTION

The box-plate for stabilizing the cervical column as proposed by the invention, is configured itself as an evident novelty allowing it to be used both to prevent and surgically treat the inestability of the cervical column after a discectomy or corporectomy performed on the cervical column, either frontally or frontal-laterally performed.

In a most specific way, the box-plate for stabilizing the cervical column of the invention, is constituted - starting from a part which is configured like a metal - plate, and an element emerging from said part, intended for occupying the space of the removed intervertebral - disk, configuring, respectively, both the plate and the the support or box, and being formed by a sole element fixed to the cervical column by means of screws, and it is to be pointed out that the support is configured like a box in the strict sense.

The zone corresponding to the plate itself presents a small profile, non-moudable and being preformed in order to be placed in an adequate way on the front face of the vertebral areas, while the support or box, i.e. the element intended for occupying the removed intervertebral disk space forms a part of the same plate, but presenting a quadrangular configuration having rounded edges.

It should be pointed out that the zone constituting the support or box emerging from the plate, presents a 10º inclination in relation to the plate in question, and it is located at the center of the plate, presenting a convenient casting in order to only configure the load edges with the aim of allowing it to be filled up with a bony graft.

The front and tear faces are closed, while the lateral ones are fully open in order to allow them to be filled up with the mentioned bony graft.

The upper and lower faces are open but presenting a screen which allows for a bony integration, but being at the same time capable of impeding the craft inserted thru the lateral faces during the implantation of the plate from emerging.

At the centre of the front face of the plate, there is a hooking element in order to fasten and handle the plate by means of an appropiate tool or a pair of pinces.

The box-plate is fastened to the front face of the vertebral bodies with the collaboration of two screws, one of them being located at the middle line of each vertebral body, these screws being of conventional spongy type, provided with a safety washer braking the screw to prevent it from moving and displacing from its fastening zone, i.e. it presents a unicortical hold configured as a safety closing washer blocking the incorporated screw, preventing it from being loose.

The support or box, presenting an adequate size, as already mentioned, is integral with the plate, having also an appropiate size, by means of a screw, the part constituting the support or box having a slightly wedged configuration, i.e. its thickness varies lightely, the upper and lower faces having the described openings, which are configured on the lines of the existence of several orifices which make possible a bony integration, but, at the same time, they prevent the graft from emerging during the positioning of the plate, which has an appropiate hooking system in order to fasten and handle the same with pinces.

The mentioned plate is provided with lateral small wings between which the invention is inserted, incorporatings a safety closing washer the function of which is to brake the screw preventing this from moving and going out.

### DESCRIPTION OF DRAWINGS

In order to complement this description and aid to a better understanding of the charactaristics of the invention, the appending drawings, which are a part of this specification, show, by way of illustrative and - non-limiting example, the following:
Figure 1 corresponds to a side elevational view, duly sectioned, of the object of the invention, relative to a box-plate for stabilizing the cervical column, showing the inclination existing between the plate itself and the support or box, this inclination being 10º.
Figure 2 shows a plan view of the object represented in figure 1, taken from the face of the part from which the support or box body emerges.
Figure 3 shows a view taken thru A-B of the object represented in figure 2.
Figure 4 corresponds to a detail of the support or box in the strict sense.
Figure 5 shows, lastly, a side elevational view of the object shown in figures 1 and 2, duly provided with the fastening screws on a vertebral body.

### DESCRIPTION OF THE PREFERRED EMBODIMENT OF THE INVENTION

From these figures, it can be seen that the box-plate for stabilizing the cervical column (1) of the invention, is constituted starting from a metal part presenting a zone (2) constituting the plate from which, thru one of its faces, a projection or support or box emerges, adopting a rectangular base configuration (2), while the projection (3) adopts a quadrangular tubular body shape, presenting a 10º inclination in relation to the plate(2), and located on the centre zone of the plate (2), being internally hollow, only presenting load edges to be filled with a bony graft in the inner zone (5).

The part so-called plate (2) presents two perforations (4) and (4'), thru which fastening screws (6) and (6') are inserted and fastened on the vertebral body (10) and (10'), the projection or support or box (3) remaining between the two vertebral bodies, being housed in the space corresponding to the removed intervertebral disk.

As mentioned in Summary of the Invention, the centre zone of the front face of the plate (2) has a hooking system for fixing and handling the plate by means of an appropiate tool or a pair of pinces, the plate (2) and the support or box attached to it (3) being fixed to the front face of the vertebral bodies (10) and (10') by means of screws (6) and (6'), these screws (6) and (6') being configured as conventional spongy elements, with unicortical hold - and a blocking screw on the head of same to prevent it from being loose.

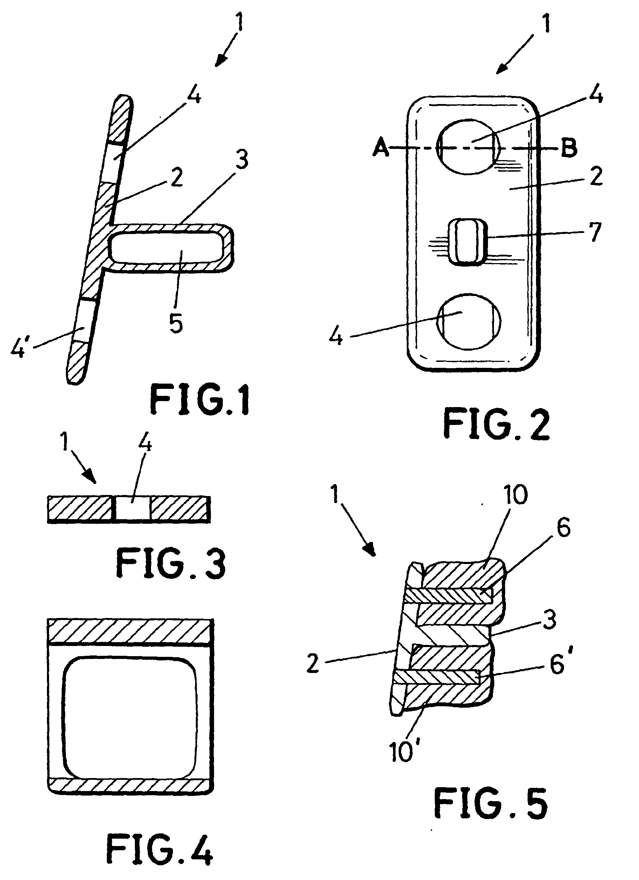

The invention integrates the conventional plate and the support or box element, i.e. the parts (2) and (3) respectively, as a whole, the support or box (3) being incorporated into the vertebral bodies (10) and (10') such as represented in figure 5, so obtaining an immediate stability, of compression belated, and a load distribution, - avoiding the loads to be only supported by the screws,the only function of which is to fix the box-plate (1) to the cervical column.

At the same time, it should be pointed out that the support or box (3) can be filled up, thru its inner zone (5) with bony graft or other osteoinhabitable material, which favours the bony fusion and the stalibilty a long time.

The box-plate (1) is made like a monoblock part, and both the box-plate (1) and the screws (6) and (6') are to be made of a titanium alloy material capable of being grafted into the human organism.

Lastly, it should be pointed out that the handling of the box-plate requires the use of a pair of fastening - pinces or other similar tool to be coupled to the centre groove (7) intended for this purpose to minimize the mobility operations.

## Claims

1. Box-plate for stabilizing the cervical column, characterized in that it is constituted starting from a monoblock body (1) made of metal material, preferably a titanium alloy, presenting a rectangular base (2), provided with perforations (4) and (4'),having, at the centre zone of the front face, a groove for incorporating a pair of fastening pinces or handling tool, presenting, at the opposite side, a hollow projection (3) constituting a support or box to be coupled into the space of the removed intervertebral - disk, while screws (6) and (6') are inserted in vertebral bodies (10) and (10') adjacent to the space of the intervertebral disk, the support or box (3) presenting, at its inner part, a hollow space (5) in which a bony graft or other osteoinhabitable material is inserted.

2. Box-plate for stabilizing the cervical column, according to claim 1, characterized in that the screws (5) and (6') are configured as spongy screws, provided with a safety closing washer for braking the screw.

3. Box-plate for stabilizing the cervical column, according to the preceding claims, characterized in that the support or box (3) presents 10º inclination in relation to the plate (2).

4. Box-plate for stabilizing the cervical column, according to the preceding claims, characterized in that the support or box (3) configures like a body having a differentiated thickness at both ends, being configured as a wedged part, having several orifices at the upper and lower faces.

5. Box-plate for stabilizing the cervical column, according to the preceding claims, characterized in that the plate (2) has lateral small wings, which are fixed by means of self-tapping screws.
